# EUROPEAN PATENT APPLICATION

(11) **EP 3 679 857 A1**
(43) Date of publication of application: **15.07.2020**
(21) Application number: 19000595.9
(22) Date of filing: 27.12.2019
(51) Int. Cl.: A61B 5/00, A61B 5/024, A61B 5/11

(54) **A DEVICE MONITORING THE EXAMINED PERSON'S BEHAVIOUR DURING THE DIAGNOSIS**

(30) Priority: 08.01.2019 PL 42852019
(71) Applicant: Politechnika Slaska, 44-100 Gliwice (PL)
(72) Inventor: Dobrowolska, Malgorzata, 40-540 Katowice (PL); Socha, Vladimir, 04022 Kosice (SK); Bereska, Damian, 44-100 Gliwice (PL); Jedrasiak, Karol, 44-190 Miko?ÓW (PL); Nawrat, Aleksander, 41-807 Zabrze (PL); Kwiatkowski, Jan, 41-933 Bytom (PL)

(57) **Abstract**

A device monitoring the examined person's behaviour during the diagnosis, in a form of glasses, comprising a frame provided with sensors, characterised in that the frame (1) comprises a tri-axial accelerometers and gyroscopes (3), outside the frame (1), on the internal side of the frame (1) electrodes (5) to measure peripheral pulse and on a support (6) of the frame (1) a camera (7). The device is powered via a micro USB (2) with the input located on the frame (1). The USB port (2) located outside the frame (1) serves as a port to send measurement data. The electrodes (5) are located on a plastic protruding element (4) which function is to fill up the space between the frame (1) and the skin of the head. Plastic protruding element (4) including the electrode (5) comprises an elastic element of a sharp approach angle towards the head, providing the optimum filling up of the space between the frame and the vault area of the head providing enough pressure of the electrodes onto the skin.

## Description

The subject of the invention is a device monitoring the examined person's behaviour during the diagnosis, intended for monitoring respondent state, during psychological examination.

During each psychological examination with psychological/assessment tests, regardless of their form which may include: paper methods (printout of workbooks, illustrations, questionnaires, worksheets, task books) and methods using modern technologies (computer software, touch screens), psychologists/researchers observe and classify the respondent behaviour. In particular, it refers to the emotions being experienced and the nature thereof (impatience, irritation, sadness, satisfaction, anxiety, etc.), cognitive processes (perception, understanding of instructions, asking questions, remembering, time of reaction, decisiveness, etc.), non-verbal communication and patient behaviour (facial expressions, gestures, etc.). The obtained data is described in the places marked in the tests. The important element, which is key to uniformity of assessment often conducted by more than one person, is unification of the method evaluating the patient state, which additionally shall enable exact mapping of the actual situation. This requires appropriate training for the psychologist/researchers. The approach applied most often results in that a human factor may have significant influence on the results obtained from the research, distorting credibility thereof. Hence, providing clarity of analysis during assessment of cognitive, behavioural and emotional aspects of the participant is an extremely important element in the art of psychology.

From patent description US20110151418A1 it is known a device intended for supporting the process of psychological assessment by obtaining, collecting and analysing of the psychological data and/or psychopathological data with the aim to select relevant treatment of the identified condition.

From patent description US3438141A it is known a device for supporting psychological assessment with the aim to present the questionnaire questions and to share the data input method as well as to collect the data obtained in testing.

Both examples use particular tests and concepts of tests focused on particular cases. New approaches to psychology are targeted at objectivisation and classification of behavioural signs that nowadays are most often assessed subjectively. These traits are mainly assessed in profiling of the subject being assessed. The object of the invention is thus to increase efficiency and provide uniformity and clarity in determining the cognitive, behavioural and emotional aspects of the individual under psychological examination.

Limitations with respect to objectivisation and classification of behavioural traits are eliminated to a great extent by monitoring participant behaviour at assessment. A device in a form of glasses comprises sensors to detect peripheral pulse on a temporal area and a tri-axial gyroscope for registration of vibrations and inclinations and tilts of a head. The mentioned sensors are a portion of a side belt attached to a glasses frame. A camera recognising a part of the face, eyes and forehead is attached to a supporting construction. A video stream from the camera is processed by an image processing technique, detected distinctive features of facial expressions together with registration of eye balls movement as well as gyroscope signals and pulse sensor values are being sent to an external storage medium of the device.

A microprocessor unit does the collection and transmit the data via the USB port and then conducts analysis of the data ad hoc or post hoc using defined classification criteria, for example, machine learning methods.

The advantage of the invention is the possibility to assess the measurement data ad-hoc and post-hoc, which enables determination of cognitive, behavioural and emotional aspects of the participant. The advantage of the invention is the possibility to use classification algorithms directly on the data measured by the device.

### The device has been presented in the preferred embodiment

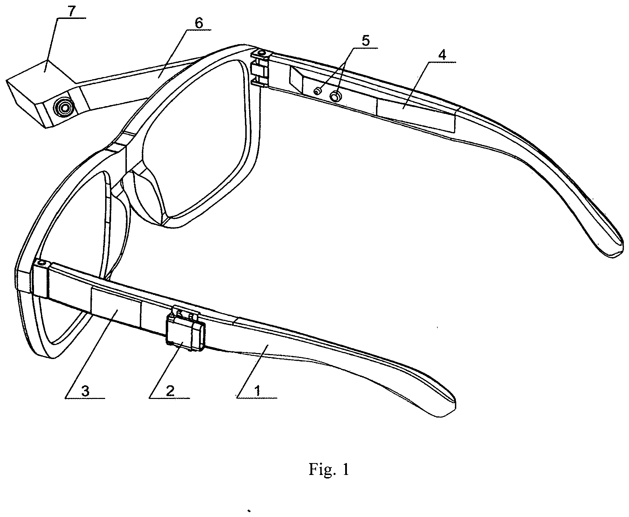

The visualisation of the device in an isometric view is shown in Fig. 1.

### Detailed description of the drawings

The device according to the invention comprises a frame 1, in a form of glasses, which serves as a supporting element for a sensory portion of the device, as well as a standard construction for a wearable type of devices. A power module 2 is located in a form of a micro USB in the device frame 1. The power module 2 serves also as a data transmission module to a PC or to a relevant data collecting and processing unit. A tri-axial gyroscope and accelerometer 3 is mounted on the frame 1. The tri-axial gyroscopes and accelerometers 3 are arranged such that to register the coordinate system of the device. The localisation of the tri-axial gyroscopes and accelerometers 3 allows to detect the movement of the head in all three axes as well as to detect vibrations. On an internal side of the frame 1 there is a two-side shaped, plastic protruding element 4 which has a direct contact with the skin of the head. A pair of dry electrodes 5 serving for differential capture of a peripheral pulse signal from a vault area of the head is on the element 4. The element 4 is designed such that the electrodes 5 are in a permanent contact with the skin.

The frame 1 has a support 6 with a wide-angle camera 7 with an adjustable focal length. All the measured signals are initially processed by internal microprocessor modules of particular sensors and sent via an integrated bus to the USB port 2.

## Claims

1. A device monitoring the examined person's behaviour during the diagnosisain a form of glasses, comprising a frame provided with sensors, **characterised in that** the frame (1) comprises a tri-axial accelerometers and gyroscopes (3), outside the frame (1), on the internal side of the frame (1) electrodes (5) to measure peripheral pulse and on a support (6) of the frame (1) a camera (7).

2. The device according to claim 1, **characterised in that** the device is powered via a micro USB (2) with the input located on the frame (1).

3. The device according to claim 1 or 2, **characterised in that** the USB port (2) located outside the frame (1) serves as a port to send measurement data.

4. The device according to any of the preceding claims, **characterised in that** the electrodes (5) are located on a plastic protruding element (4) which function is to fill up the space between the frame (1) and the skin of the head.

5. The device according to claim 4, **characterised in that** the electrodes (5) are dry electrodes.

6. The device according to claim 4 or 5, **characterised in that** the plastic protruding element (4) including the electrode (5) comprises an elastic element of a sharp approach angle towards the head, providing the optimum filling up of the space between the frame and the vault area of the head providing enough pressure of the electrodes onto the skin.

7. The device according to any of the preceding claims, **characterised in that** the frame (1) comprises the support (6) of a permanent or telescopic construction wherein the camera (7) is mounted.

8. The device according to any of the preceding claims, **characterised in that** each sensor comprises a microprocessor unit designed to preliminary process data and send via an integrated bus to the USB port (2).
